(19) Europäisches Patentamt
European Patent Office
Office européen des brevets

(11) EP 3 238 758 A1

(12) EUROPEAN PATENT APPLICATION
published in accordance with Art. 153(4) EPC

(43) Date of publication:
01.11.2017 Bulletin 2017/44

(51) Int Cl.:
A61M 1/18 (2006.01)
B01D 61/28 (2006.01)
B01D 63/00 (2006.01)
B01D 63/02 (2006.01)

(21) Application number: 15873325.3

(22) Date of filing: 25.12.2015

(86) International application number:
PCT/JP2015/086356

(87) International publication number:
WO 2016/104757 (30.06.2016 Gazette 2016/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 25.12.2014 JP 2014262758

(71) Applicant: Asahi Kasei Medical Co., Ltd.
Chiyoda-ku
Tokyo 101-8101 (JP)

(72) Inventors:
• YOKOYAMA, Hiroya
Tokyo 101-8101 (JP)
• NISHIZAWA, Kazuki
Tokyo 101-8101 (JP)
• SUKEGAWA, Takeshi
Tokyo 101-8101 (JP)
• YAGINUMA, Yoshihito
Tokyo 101-8101 (JP)

(74) Representative: dompatent von Kreisler Selting Werner -
Partnerschaft von Patent- und Rechtsanwälten mbB
Deichmannhaus am Dom
Bahnhofsvorplatz 1
50667 Köln (DE)

(54) **HEMODIAFILTER AND HEMODIAFILTRATION DEVICE**

(57) A hemodiafilter includes a cylindrical container and a bundle of hollow fiber membranes packaged in the cylindrical container, wherein both ends of the hollow fiber membranes are potted to both ends of the cylindrical container with curing resin, having opening end faces on both ends of the cylindrical container, the hollow fiber membrane has a crimped shape, the hollow fiber membrane bundle in the cylindrical container has a filling ratio F(%) of 53% to 63%, a distance L(mm) between both opening end faces of the hollow fiber membrane is 325 mm or less, and an effective membrane area $A(m^2)$ of the hollow fiber membrane bundle and an opening ratio X(%) on an opening end face of the hollow fiber membrane bundle satisfy $18 + 2 \times A < X < 28 + 2 \times A$.

[Fig 1]

$d_1$

$d_0$

EP 3 238 758 A1

**Description**

Technical Field

The present invention relates to a hemodiafilter and a device including the hemodiafilter.

Background Art

Hemodiafiltration is a therapy that removes uremic substances over a wide molecular weight range from small molecular weight substances to low molecular weight protein by using the effects of filtration and dialysis (diffusion). In a method of this therapy, a high rate of filtration is performed from blood to dialysate passing through a hemodiafilter, and then an excessive reduction in body fluid volume by the filtration is replaced with an infused electrolyte solution (substitution fluid). This method can remove uremic substances over a wide molecular weight range from small molecular weight substances to low molecular weight protein, providing a treatment having a function similar to a human glomerulus.

It is reported that hemodiafiltration has been effective for arthralgia, pruritus, insomnia, nervousness, restless legs syndrome, and so on. It is also understood that hemodiafiltration reduces the risk of amyloidosis.

Hemodiafiltration is categorized as, for example, an off-line type (bottle type), an on-line type, and a push-and-pull type. Off-line hemodiafiltration is a classic method in which an infusion is used as a substitution fluid. A glass bottle or a soft bag is filled with 1 or 2 liters of infusion that is an infusion for hemodiafiltration. An excessive reduction in body fluid volume by the hemodiafilter is replaced with an infused electrolyte solution (substitution fluid). Generally, 5 to 20 liters of a commercial substitution fluid for hemodiafiltration is used at a time.

In on-line hemodiafiltration, a drip (replenisher) is not used and a fluid separated from a fluid for dialysis is directly used as a replenisher. On-line hemodiafiltration can advantageously provide a substitution for a larger volume of replenisher than ordinary hemodiafiltration. Moreover, only patients having specific diseases can be insured in off-line hemodiafiltration, whereas any dialysis patient can be insured in on-line hemodiafiltration because remuneration for medical treatment was revised in 2012. Thus, the number of dialysis facilities for on-line hemodiafiltration has increased after the revision of the remuneration for medical treatment.

Since a substitution fluid has a small volume in conventional off-line hemodiafiltration, hemodiafilters (hemodialyzer) used for hemodiafiltration are shaped like hollow-fiber membrane modules used for hemodialysis but any original designs have not been invented for hemodiafiltration modules.

For example, a module for hemodiafiltration has been invented to improve the fractionation characteristics of $\beta2MG$ and albumin (Patent Literature 1). In the case of a large container length or hollow fiber length, a pressure increases near an inlet of blood.

Patent literature 2 discloses an invention relating to the shape of a hollow-fiber extracorporeal circulation module including hemodiafiltration. The invention collectively describes hemodiafiltration and hemofiltration and but does not discuss an original shape based on the principles of hemodiafiltration. Actually, the infusion of dialysate is not taken into consideration, and hollow fiber membranes bundled with a high filling ratio may lead to a narrow dialysate passage.

Citation List

Patent Literature

Patent Literature 1: Japanese Patent Laid-Open No. 2009-78121
Patent Literature 2: Japanese Patent No. 4992104

Since the number of on-line hemodiafiltration treatments has rapidly increased in recent years, a large volume of substitution fluid is added to removed blood of a patient. This may increases a flow rate of blood and a pressure loss near an inlet of blood, thereby increasing a blood inlet pressure to pose a risk of interrupting a treatment.

In hemodiafiltration, it is expected to increase the amount of removed blood substances more than in hemodialysis. Thus, the flow rate of dialysate tends to be increased using a module having a large membrane area. This may increase a pressure loss near the dialysate to pose a risk of accelerating membrane deterioration.

Summary of Invention

Technical Problem

In view of the problems that have not been solved in the related art, an object of the present invention is to provide a hemodiafilter that can keep pressure losses at low values on both of a blood side and a dialysate side during hemodiafiltration.

Solution to Problem

As a result of a thorough examination for solving the problems, the present inventors have found that, by using hollow fiber membranes having crimped shapes and packing them in a cylindrical container such that specific conditions are satisfied concerning: a membrane bundle filling ratio F(%); and an opening ratio X(%) of an end face having openings of a bundle of hollow fiber membranes or an opening ratio Y(%) of a blood flow path area on one end face of curing resin used for potting processing, pressure losses in a hemodiafilter can be kept at low values on both the blood side and the dialysate side. Thus, the present inventors have completed the present invention.

Specifically, the present invention relates to a hemodiafilter and a hemodiafiltration device including the hemodiafilter.

[1] A hemodiafilter including a cylindrical container and a bundle of hollow fiber membranes packaged in the cylindrical container,

> wherein both ends of the hollow fiber membranes are potting processed at both ends of the cylindrical container with curing resin, and have end faces having an opening on both ends of the cylindrical container,
> the hollow fiber membrane has a crimped shape,
> a filling ratio F(%) of hollow fiber membrane bundle in the cylindrical container is 53% or more and 63% or less,
> a distance L(mm) between both end faces having an opening of the hollow fiber membrane is 325 mm or less, and
> an effective membrane area $A(m^2)$ of the hollow fiber membrane bundle and an opening ratio X(%) of an end face having openings of the hollow fiber membrane bundle satisfy expression (1) below:

$$18 + 2 \times A < X < 28 + 2 \times A \qquad (1)$$

[2] A hemodiafilter including a cylindrical container and a bundle of hollow fiber membranes packaged in the cylindrical container,

> wherein both ends of the hollow fiber membranes are potting processed at both ends of the cylindrical container with curing resin, and have end faces having an opening on both ends of the cylindrical container,
> the hollow fiber membrane has a crimped shape,
> a filling ratio F(%) of the hollow fiber membrane bundle in the cylindrical container is 53% or more and 63% or less,
> a distance L(mm) between both end faces having an opening of the hollow fiber membrane is 325 mm or less, and
> the filling ratio F(%) of the hollow fiber membrane bundle and an opening ratio Y(%) of a blood flow path area on an end face of the curing resin satisfy expression (a) below:

$$6.4 + 0.2 \times F < Y < 14.4 + 0.2 \times F \qquad (a)$$

[3] The hemodiafilter according to [1] or [2], wherein the effective membrane area A is 1.6 $m^2$ or more.
[4] The hemodiafilter according to any one of [1] to [3], wherein an inside diameter of the hollow fiber membrane B is 195 $\mu$m or more and 205 $\mu$m or less.
[5] The hemodiafilter according to any one of [1] to [4], wherein a ratio L/D is 5.5 or more and 9.0 or less where L(mm) is a distance between both end faces having an opening of the hollow fiber membrane bundle and D(mm) is an inside diameter of the cylindrical container.
[6] The hemodiafilter according to any one of [1] to [5], wherein the cylindrical container has a dialysate inlet and a dialysate outlet, and
the cylindrical container has a portion where the inside diameter of the cylindrical container D increases in the longitudinal direction from the dialysate inlet to the dialysate outlet.

[7] The hemodiafilter according to any one of [1] to [6], wherein the cylindrical container has the dialysate inlet and the dialysate outlet, and

the cylindrical container has a portion where the inside diameter of the cylindrical container D decreases in the longitudinal direction from the dialysate inlet to the dialysate outlet.

[8] The hemodiafilter according to any one of [1] to [7], wherein the inside diameter of the cylindrical container once decreases and then increases in the longitudinal direction from the dialysate inlet to the dialysate outlet.

[9] The hemodiafilter according to any one of [1] to [8], wherein an average taper "a" of the inside diameter of the cylindrical container in the longitudinal direction at the dialysate outlet side is from 0.7 to 1.5%.

[10] The hemodiafilter according to any one of [6] to [9], wherein the position where the inside diameter of the cylindrical container is at a minimum is located at the dialysate inlet from a halfway point between the dialysate inlet and the dialysate outlet.

[11] The hemodiafilter according to any one of [8] to [10], wherein a ratio L2/L3 is from 0.5 to 0.99 where L2 is a length from a dialysate outlet side end of the body of the cylindrical container to a point where the minimum inside diameter is at a minimum, and L3 is a length of the body of the cylindrical container.

[12] The hemodiafilter according to any one of [6] to [11], wherein the cylindrical container further includes a blood inlet and a blood outlet, the blood outlet being disposed at the dialysate inlet side, and the blood inlet being disposed at the dialysate outlet side in the longitudinal direction.

[13] A hemodiafiltration device including: the hemodiafilter according to any one of [1] to [12]; a blood flow path and a dialysate feed flow path that are connected to the hemodiafilter; and a substitution fluid feed flow path branching from the dialysate feed flow path,

wherein the substitution fluid feed flow path is connected to the blood flow path upstream of the blood inlet and/or downstream of the blood outlet of the hemodiafilter.

[14] The hemodiafiltration device according to [13], further including:

a blood pump that allows blood to flow into the hemodiafilter through the blood flow path;
a dialysate feed pump that supplies dialysate into the hemodiafilter through the dialysate feed flow path; and
a substitution fluid pump that feeds a substitution fluid to the blood flow path through the substitution fluid feed flow path.

Advantageous Effects of Invention

The hemodiafilter of the present invention can keep pressure losses at low values on both of the blood and dialysate sides to enable on-line hemodiafiltration.

Among types of on-line hemodiafiltration, the hemodiafilter of the present invention is specifically effective for pre-dilution in which a substitution fluid is replenished before patient's blood flows into the hemodiafilter. However, the hemodiafilter of the present invention is also effective for a combination use of pre-dilution and post-dilution in which a substitution fluid is replenished after patient's blood passes through the hemodiafilter.

This reduces a positive pressure at the blood inlet and a negative pressure at the dialysate outlet, thereby achieving stable medical treatment and suppressing a reduction in filterability.

Brief Description of Drawings

[Figure 1] Figure 1 is an explanatory drawing of an opening ratio X of the end face having openings of a hollow-fiber membrane bundle (the area of the hollow-fiber membrane bundle) and an opening ratio Y of a blood flow path area on an end face of curing resin.
[Figure 2] Figure 2 is an explanatory drawing showing an average taper "a" in the longitudinal direction of the inside diameter of the cylindrical container. Description of Embodiment

An embodiment of the present invention (Hereinafter, will be referred to as "the present embodiment") will be specifically described below.

The present embodiment is exemplarily described and the present invention is not limited to the following description. The present invention can be optionally modified within the scope of the invention.

The material of a hollow fiber membrane of a hemodiafilter is not limited to a specific material. Any hollow-fiber membrane materials conventionally used for dialyzers can be used. For example, the material may be a cellulosic polymer, a polysulfone polymer, a polyacrylonitrile based polymer, a polymethyl methacrylate polymer, a polyvinyl polymer containing an ethylene-vinylalcohol copolymer, a polyamide polymer, a polyester polymer, or a polyolefin polymer.

The hollow fiber membrane can be manufactured according to a known technique.

The hollow fiber membrane may be formed by blending a hydrophilic polymer to provide hydrophilicity for a hydrophobic polymer. The hydrophilic polymer may be, for example, polyvinyl pyrrolidone (PVP), polyethylene glycol, polyvinyl alcohol, or polypropylene glycol. In view of the effect of hydrophilization and safety, polyvinyl pyrrolidone is particularly preferable but the present invention is not particularly limited to these materials.

In the production of a blend membrane of a hydrophobic polymer and a hydrophilic polymer, these polymers are first dissolved in a common solvent to prepare a spinning solution.

If the hydrophilic polymer is PVP, the common solvent may be, for example, a solvent of dimethylacetamide (Hereinafter will be referred to as DMAC), dimethyl sulfoxide, N-methyl-2-pyrrolidone, dimethylformamide, sulfolane, or dioxane or a mixed solvent of at least two of these materials. In order to control the pore size of a target hollow fiber membrane, water or the like may be added to the spinning solution.

In the production of the hollow fiber membrane, the spinning solution is preferably ejected into the air from the orifice of a tube-in-orifice spinneret concurrently with a bore liquid that is ejected from a tube to coagulate the spinning solution.

The bore liquid may be water or a coagulating solution mainly composed of water. The composition of the liquid may be determined according to the permeability of the target hollow fiber membrane. Generally, a mixed solution of water and a solvent used for a spinning solution is preferably used. For example, a DMAC solution of from 0 to 65 mass% is used.

The spinning solution ejected with the bore liquid from the spinneret is passed through an air gap portion, is introduced and immersed into a coagulating bath, which is located under the spinneret and whose content is mainly composed of water, so as to be completely coagulated. After a cleaning process and so on, the hollow fiber membrane in a wet state is wound to obtain a hollow fiber membrane bundle by a winder, and then the fiber membrane is dried. Alternatively, after the cleaning process, the fiber membrane may be dried in a drier to obtain a hollow fiber membrane bundle.

The hollow fiber membrane contained in the hemodiafilter of the present embodiment has a crimped shape. If a hollow fiber membrane bundle is packaged into a cylindrical container with a filling ratio F of 53% to 63% as will be discussed later, hollow fiber membranes are preferably crimped so as to prevent the membrane bundle from being fixed to an inner periphery away from the center of the cylindrical container to prevent blood retention or a biased dialysate flow that is likely to occur in a header during hemodiafiltration.

A method of crimping a hollow fiber membrane is not particularly limited. For example, in a gear system, fibers pressed between two gears continuously rotating with mating gears are simultaneously or successively heated to fix the crimp of the fibers. In other known methods, fibers are properly stretched at room temperature so as to obtain crimped fibers with less flat or deformed shapes, fibers wound around a bobbin or the like are heated at 50°C or higher to fix the crimp of the fibers, or continuous fibers transported so as to snake between multiple fiber guides running at regular intervals are heat-set through heat treatment.

The pitch and amplitude of the crimp are not particularly limited. For example, crimp preferably has a pitch of about from 0.1 to 2.0 cm and an amplitude of about from 0.2 to 0.8 mm and more preferably has a pitch of about from 0.4 to 0.8 cm and an amplitude of about from 0.4 to 0.6 mm.

(Filling ratio F of a hollow fiber membrane bundle)

In the present embodiment, a bundle of hollow fiber membranes (hollow fiber membrane bundle) is fixed to the cylindrical container by potting processing both ends of hollow fiber membranes to both ends of the cylindrical container with curing resin (resin is injected between the hollow fiber membrane bundle and the container and between each hollow fiber membrane with the hollow fiber membrane bundle being disposed in the cylindrical container). When hollow portions on both ends of the hollow fiber membranes are filled with curing resin during potting processing, the portions are cut so as to form end faces having an opening on both ends of the cylindrical container (to make the end faces of the hollow fiber membranes have openings).

The filling ratio F of the hollow fiber membrane bundle in the cylindrical container needs to be 53% or more and 63% or less. Preferably, the filling ratio F is 55% or more and 60% or less.

The filling ratio F of 63% or less can provide a wide flow path for dialysate, thereby suppressing a pressure loss on the dialysate side.

The filling ratio F of 53% or more can suppress the amount of used curing resin serving as a potting material and set an opening ratio X, which will be discussed later, of an end face within a proper range, thereby suppressing a pressure loss on the blood side. When the filling ratio is smaller than 53%, it is difficult to increase the end-face opening ratio on an end of the hollow fiber membrane, which will be discussed later.

The filling ratio F(%) of a hollow fiber membrane bundle is the percentage of the ratio of the total of the cross-sectional areas of hollow fiber membrane portions relative to the cross-sectional area of the cylindrical container of the hemodiafilter (the sum of the cross-sectional areas of the hollow fiber membrane portions/the cross-sectional area of the cylindrical container).

The cross-sectional area of the cylindrical container of the hemodiafilter is a net inner area (the cross-sectional area of a space in the container). If the cylindrical container is circular in cross section, the cross-sectional area is calculated from an inside diameter D of the cylindrical container of the hemodiafilter. If the cylindrical container of the hemodiafilter have different diameters in the longitudinal direction of the container, the inside diameter of the container is a value at the center of the container in the longitudinal direction. If the cylindrical container is not circular in cross section, the inside diameter is the converted value of the diameter of a perfect circle having the same cross-sectional area.

The total cross-sectional area of the hollow fiber membrane portions is a value calculated by multiplying a circular area having a diameter equal to an outside diameter (C) (will be discussed later) of the hollow fiber membrane of the hemodiafilter by the number of hollow fiber membranes. For example, if spacer yarns are inserted between hollow fiber membranes (thin fibers disposed between hollow fiber membranes to rectify dialysate), the total cross-sectional area of hollow fiber membrane portions is a value determined by adding the total cross-sectional area of insertions (e.g., spacer yarns) other than hollow fiber membranes (a value calculated by multiplying the area of a circle having a diameter equal to the outside diameter of the spacer yarn by the number of spacer yarns).

(The inside diameter, the outside diameter, and the thickness of the hollow fiber membrane)

The inside diameter, the outside diameter, and the thickness of the hollow fiber membrane can be measured from the cross section of the end faces of a hollow fiber membrane bundle in the hemodiafilter, which is potting processed with curing resin. The inside diameter, the outside diameter, and the thickness can be measured as follows:

The shortest and longest diameters of the cross sections of hollow fiber membranes on the cross section of a hollow fiber membrane bundle are observed and measured through a microscope (e.g., Digital Microscope VH8000 (KEYENCE CORPORATION)). An outside diameter and an inside diameter are measured in two directions, that is, the shortest diameter direction and the longest diameter direction on each cross section of a hollow fiber membrane (a cross section of an extremely deformed hollow fiber membrane is excluded from the measurement), and the calculated mean values thereof are determined as inside and outside diameters of the cross section of a hollow fiber membrane. A thickness of the hollow fiber membrane on the cross section is calculated from the equation of (outside diameter - inside diameter)/2. On any ten cross sections, the inside and outside diameters of hollow fiber membranes are measured and the thicknesses of the hollow fiber membranes are calculated as described above. Mean values on the ten cross sections are determined as the outside diameter (C), the inside diameter (B), and the thickness of a hollow fiber membrane in the hemodiafilter. If different values are obtained for both ends of the hollow fiber membrane bundle, the mean value of the values obtained from both cross sections is used.

(Distance L between end faces having an opening)

A distance L between both end faces having an opening of a hollow fiber is a distance from one end face having an opening to the other end face having an opening of a hollow fiber membrane fixed with a potting material (curing resin) in the hemodiafilter. The distance L between both end faces having an opening is not the length of a hollow fiber membrane but a distance between both end faces having an opening of a hollow fiber membrane in the hemodiafilter. Thus, the distance L is a distance between both end faces having an opening of the hollow fiber membrane, even when a hollow fiber membrane fixed with a potting material in the hemodiafilter has slackness, such slackness is discarded.

The distance L between both end faces having an opening of a hollow fiber membrane (a distance between end faces having an opening on both ends) in the hemodiafilter of the present embodiment is 325 mm or less. The distance L is more preferably 310 mm or less and is most preferably 290 mm or less. By setting the distance L between both end faces having an opening of a hollow fiber membrane at 325 mm or less, a distance for blood to pass through in the hollow fiber membrane becomes appropriate, thereby allowing an increase in pressure loss on the blood side to be prevented. Moreover, a flow path on the dialysate side has a proper length, suppressing a pressure loss on the dialysate side.

The lower limit of the distance L between both end faces having an opening of a hollow fiber membrane is not particularly limited but is preferably 200 mm or more and more preferably 240 mm or more in view of mountability to a dialyzer.

(Curing resin)

Examples of curing resin used for potting processing both ends of a hollow fiber membrane bundle include, for example, polyurethane resin, epoxy resin, or silicon resin. The material of curing resin is not particularly limited.

(Effective membrane area A of a hollow fiber membrane bundle)

The effective membrane area of a hollow fiber membrane bundle included in the hemodiafilter is not particularly limited. However, it is expected that the amount of removed substances in blood can be increased using a large volume of substitution fluid in the hemodiafiltration, and thus the effective membrane area is preferably 1.6 $m^2$ or more and more preferably 2.0 $mm^2$ or more.

The effective membrane area A ($m^2$) is calculated from the product of an effective length l (mm), an inside diameter B ($\mu$m), circular constant $\pi$, and the number n of hollow fiber membranes included in a hollow fiber membrane bundle (n $\times$ l $\times$ $\pi$ $\times$ B $\times$ $10^{-9}$).

In this case, the effective length l of the hollow fiber membrane is the length of a potion actually acting as a semipermeable membrane of a hollow fiber membrane through filtration and dialysis. The effective length does not include a portion sealed with curing resin on both ends of the hollow fiber membrane. Specifically, the effective length l is the length of a portion between curing resin of the hollow fiber membrane. If the effective length varies among hollow fiber membranes, the mean value of all the hollow fiber membranes included in the hollow fiber membrane bundle is used.

(Opening ratio X of the end face having openings of a hollow fiber membrane bundle)

In a hemodiafilter according to an aspect of the present embodiment, the effective membrane area A ($m^2$) of a hollow fiber membrane bundle and an opening ratio X(%) of the end face having an opening of a hollow fiber membrane bundle satisfy expression (1) below:

$$18 + 2 \times A < X < 28 + 2 \times A \qquad (1)$$

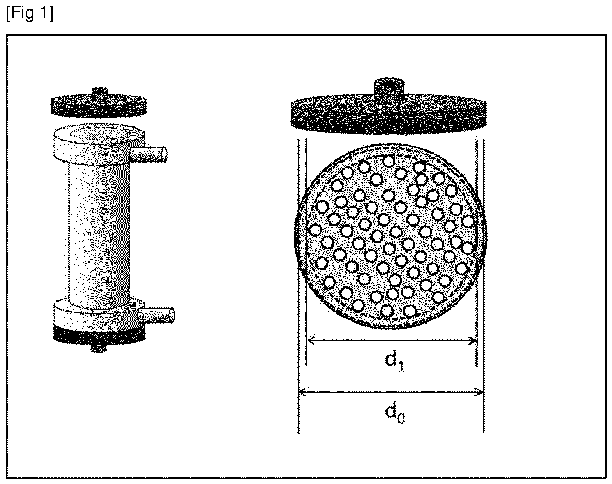

The opening ratio X(%) of the end face having openings of the hollow fiber membrane bundle is the ratio of an internal area of the hollow fiber membranes (the total of areas of internal portions of the hollow fiber membranes) relative to an area of the hollow-fiber-membrane bundle (an outer area (a substantially circular region surrounded by a line connecting hollow fiber membranes located on the outermost periphery of the hollow fiber membrane bundle on one end face of curing resin) on one end of a potting processed portion of the hollow fiber membrane bundle (see Figure 1)). The opening ratio X(%) is calculated as follows:

An opening ratio X(%) of an end face of a hollow fiber membrane bundle =

(the internal area of the hollow fiber membranes / the area of the hollow fiber

membrane bundle) $\times$ 100

The area of the hollow fiber membrane bundle area is determined as follows. Since the external shape of a potting processed portion of a hollow fiber membrane bundle packaged in the hemodiafilter is substantially circle, a diameter $d_1$ (Figure 1) of the external shape is measured in any five directions by using vernier calipers. A diameter is not measured at a location (in a direction) where a hollow fiber membrane is extremely apart from the external shape. An area of a circle having a diameter equal to the average value of the diameters measured in the five directions described above is calculated, and the area is determined as an area of the hollow fiber membrane bundle ($mm^2$).

The internal area of the hollow fiber membranes is calculated by the following expression:

The internal area of the hollow fiber membranes ($mm^2$) = n $\times$ $\pi \times$ {B/2}$^2$ $\times$ $10^{-6}$

where B($\mu$m) is the inside diameter of the hollow fiber membrane and n is the number of hollow fiber membranes contained in a hollow fiber membrane bundle of the hemodiafilter.

When the opening ratio X of the end face having openings of the hollow fiber membrane bundle exceeds (18 + 2 $\times$ A), a pressure loss can be suppressed on the blood side. When the opening ratio X of the end face having openings of a hollow fiber membrane bundle is less than (28 + 2 $\times$ A), a filling ratio in the cylindrical container of the hemodiafilter can be set in a proper range so as to prevent an increase in pressure loss on the dialysate side. When the opening ratio X of the end face having openings of a hollow fiber membrane bundle becomes (28 + 2 $\times$ A) or higher, it is difficult to reduce the filling ratio of the membrane bundle.

(Opening ratio Y of a blood flow path area on one end face of curing resin)

In the hemodiafilter according to another aspect of the present embodiment, the filling ratio F(%) of the hollow fiber membrane bundle and the opening ratio Y(%) of the blood flow path area on one end face of curing resin for potting processing satisfy the following expression (a).

$$6.4 + 0.2 \times F < Y < 14.4 + 0.2 \times F \text{ (a)}$$

The opening ratio Y(%) of the blood flow path area on the end face of curing resin is the ratio of the internal area of the hollow fiber membranes (the total area of the internal parts of the hollow fiber membranes) relative to the area of a portion (see Figure 1) serving as a blood flow path (in contact with blood) during the use of the hemodiafilter of the end face of curing resin (the end face formed of curing resin used for potting processing) at the blood inlet side. The opening ratio Y(%) can be calculated as follows:

The opening ratio Y(%) of the blood flow path area on the end face of curing resin = (the internal area of hollow fiber membranes/the area of the end face of curing resin) × 100

In the hemodiafilter of the present embodiment in which both ends of a hollow fiber membrane are fixed to both ends of the cylindrical container by potting processing with curing resin, a portion serving as a blood flow path on one end face of curing resin during the use of the hemodiafilter is naturally determined depending on the shape or configuration and the mounting method of a header etc. attached to the end face of curing resin. Typically, the portion is slightly smaller than whole area of the end face of curing resin (see Figure 1).

The area of the blood flow path on the end face of curing resin is determined as follows. Since the external shape of the blood flow path on the end face of curing resin is substantially circle, the diameter $d_0$ (Figure 1) of the external shape is measured in any five directions by using vernier calipers. The area of a circle having a diameter (mm) equal to the average value of diameters measured in the five directions described above is calculated, and the area is determined as the area of the blood flow path on the end face of curing resin $(mm^2)$.

A hollow-fiber-membrane internal area is calculated by the following expression:

$$\text{Hollow-fiber-membrane internal area } (mm^2) = n \times \pi \times \{B/2\}^2 \times 10^{-6}$$

where $B(\mu m)$ is the inside diameter of a hollow fiber membrane and n is the number of hollow fiber membranes contained in a hollow fiber membrane bundle of the hemodiafilter.

By setting the opening ratio Y of the blood flow path area on the end face of curing resin larger than (6.4 + 0.2 × F), a pressure loss can be suppressed on the blood side. By setting the opening ratio Y of the blood flow path area on the end face of curing resin falls smaller than (14.4 + 0.2 × F), a filling ratio in the cylindrical container of the hemodiafilter can be set in an appropriate range so as to prevent an increase in pressure loss on the dialysate side. When the opening ratio Y of the blood flow path area on the end face of curing resin is (14.4 + 0.2 × F) or higher, it is difficult to reduce the filling ratio of the membrane bundle.

The inside diameter B of a hollow fiber membrane is not particularly limited. An excessively small inside diameter reduces the opening ratio X of the end face having openings of the hollow fiber membrane bundle. Thus, the inside diameter B is preferably at 195 $\mu m$ or more if the effective membrane area A is set within an ordinary range (about 1.0 $m^2$ or more and 3.2 $m^2$ or less). Moreover, an extremely large inside diameter excessively increases the filling ratio of the hollow fiber membrane bundle in the cylindrical container. Thus, the inside diameter is preferably 250 $\mu m$ or less, is more preferably 230 $\mu m$ or less, and is most preferably 205 $\mu m$ or less.

The thickness of a hollow fiber membrane is not particularly limited.

The hollow fiber membrane having a small thickness exhibits high material permeability but has low strength. Thus, the thickness of the hollow fiber membrane is preferably 20 $\mu m$ or more and is more preferably 30 $\mu m$ or more in view of the strength of the hollow fiber membrane.

The hollow fiber membrane preferably has a thickness of 55 $\mu m$ or less and more preferably has a thickness of 50 $\mu m$ or less in view of membrane permeability, the filling ratio of the hollow fiber membrane bundle, and ease of insertion into a case.

In the hemodiafilter of the present embodiment, a distance L(mm) between the end faces having an opening of the hollow fiber membrane/the inside diameter D(mm) of the cylindrical container is not particularly limited. Preferably, L/D is from 5.5 to 9.0.

In the hemodiafilter of the present embodiment, the cylindrical container may have a dialysate inlet and a dialysate outlet, and its inside diameter may increase in the longitudinal direction from the dialysate inlet side to the dialysate outlet side. This configuration realizes a large-volume space in the cylindrical container near the dialysate outlet, thereby effectively prevents an increase in pressure loss on the dialysate side, particularly around the dialysate outlet, during hemodiafiltration that increases a flow rate at the dialysate outlet relative to the dialysate inlet.

In the hemodiafilter of the present embodiment, the cylindrical container may have a dialysate inlet and a dialysate outlet, and its inside diameter may decrease in the longitudinal direction from the dialysate inlet side to the dialysate outlet side. This configuration can accelerate a flow of dialysate from the dialysate inlet into a hollow fiber membrane bundle during hemodiafiltration, thereby allowing the effective filtration area of the hollow fiber membrane bundle to be utilized effectively.

In the hemodiafilter of the present embodiment, preferably, the cylindrical container has a dialysate inlet and a dialysate outlet with an inside diameter decreasing once and then increasing in the longitudinal direction from the dialysate inlet to the dialysate outlet. This configuration can achieve the effect of accelerating a flow of dialysate into a hollow fiber membrane bundle and the effect of obtaining a sufficient space volume in the cylindrical container near the dialysate outlet at the same time. Moreover, in a cylindrical container having an inside diameter that simply increases or decreases, the inclination angle of a taper is limited in consideration of container molding. In a structure having an inside diameter that decreases once and then increases, a taper with a higher gradient can be obtained. Thus, the effect can be sufficiently produced.

In the hemodiafilter of the present embodiment, if the inside diameter of the cylindrical container decreases once and then increases in the longitudinal direction, a taper "a" (an average taper in the longitudinal direction of the inside diameter) at the dialysis outlet side is preferably 0.7 to 1.5%. The taper "a" is further preferably 0.8% or higher, is more preferably 0.9% or higher, and particularly preferably 1.0% or higher. The taper "a" of less than 0.7% is not preferable because a sufficient space volume cannot be obtained in the cylindrical container at the dialysate outlet. The taper "a" larger than 1.5% is not preferable because the barycenter of the cylindrical container deviates from the center and loses the balance of a hollow fiber membrane module during centrifugal molding, causing a manufacturing defect.

Here, an average taper "a" in the longitudinal direction of the inside diameter of the cylindrical container at the dialysate outlet side is the ratio of a difference between a maximum value of the inside diameter and a minimum value at the dialysis outlet side relative to a length from one end of the body of the cylindrical container at the dialysate outlet side to the part with a minimum inside diameter.

The body of the cylindrical container is a part having a smooth outer peripheral with no protrusion between a dialysate inlet (port) and a dialysate outlet (port).

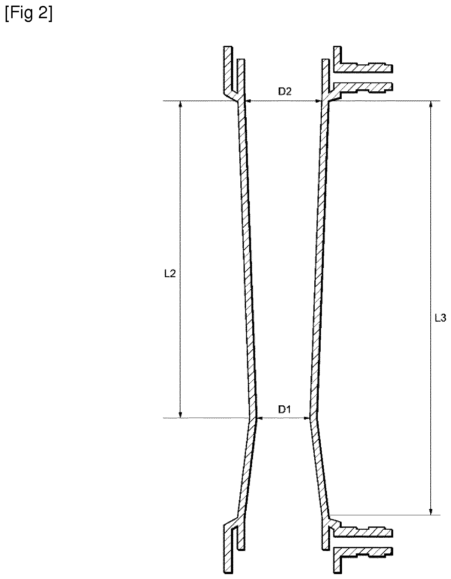

For example, the average taper "a" in the longitudinal direction of the inside diameter of the cylindrical container at the dialysate outlet side in Figure 2 is defined by the following expression:

$$a(\%) = 100 \times (D2 - D1)/L2$$

where D2(mm) is the maximum value and D1 (mm) is the minimum value of the inside diameter of the body of the cylindrical container at the dialysis outlet side, and L2(mm) is a length from the dialysate outlet end of the body to the point having an inside diameter of D1.

If the inside diameter of the cylindrical container decreases once and then increases in the longitudinal direction from the dialysate inlet to the dialysate outlet, the cylindrical container may have an asymmetric shape in the longitudinal direction such that the position having a minimum inside diameter is located at the dialysate inlet side from the halfway point between the dialysate inlet and the dialysate outlet. The configuration with the position having the minimum inside diameter located nearer to the dialysate inlet reduces the taper of the cylindrical container at the dialysate outlet side (reduces an inclination at the outlet side) and allows certain longitudinal regions of the cylindrical container to receive a pressure which is otherwise applied only to the area of the dialysate outlet. This can reduce a pressure locally applied to the area of the dialysate outlet, equalize the flow rates of dialysate in the longitudinal direction during hemodiafiltration, and suppress a rapid increase in flow rate at the dialysate outlet side. Thus, a rapid negative pressure can be reduced at the dialysate outlet side.

In the hemodiafilter of the present embodiment, the ratio of the length L2 to a length L3 of the body of the cylindrical container (L2/L3) is preferably 0.5 to 0.99, the length L2 is a length from one end of the body at the dialysate outlet side to the point having a minimum inside diameter. The ratio is more preferably 0.6 to 0.95 and is particularly preferably 0.75 to 0.9. If L2/L3 is less than 0.5, a change of the flow rate of dialysate increases in the longitudinal direction during hemodiafiltration. If L2/L3 is larger than 0.99, the effect of accelerating a flow of dialysate from the dialysate inlet into a hollow fiber membrane bundle is reduced.

In the hemodiafilter of the present embodiment, the cylindrical container may preferably further includes a blood inlet and a blood outlet. The dialysate inlet is preferably disposed near the blood outlet and the dialysate outlet is preferably disposed near the blood inlet in the longitudinal direction. With this configuration, blood and dialysate flow opposite to each other in the longitudinal direction of the cylindrical container, thereby improving blood filtration efficiency.

A pressure loss on the blood side is calculated by expression (2) below:

$$\text{Pressure loss on blood side} = \text{pressure at the blood inlet side} - \text{pressure at the blood outlet side} \qquad (2)$$

The pressure at the blood inlet side and the pressure at the blood outlet side are pressures measured during blood circulation by using a pressure detector that is attached via a branched pipe to a blood flow path near the inlet and the outlet of the hemodiafilter, respectively.

The value of the pressure loss on the blood side depends on a blood viscosity and a blood flow rate.

In the case of bovine blood conditions and a blood flow rate that are used in the evaluation tests of the following examples, a pressure loss on the blood side is preferably less than 150 mmHg.

A pressure loss on the dialysate side is calculated by equation (3) below:

$$\text{Pressure loss on dialysate side} = \text{pressure at the dialysate inlet} - \text{pressure at the dialysate outlet} \qquad (3)$$

The dialysate inlet pressure and the dialysate outlet pressure are pressures measured during blood circulation by using a pressure detector that is attached via a branched pipe to a dialysate flow path near the inlet and the outlet of the hemodiafilter, respectively.

The value of the pressure loss on the dialysate side depends on a dialysate viscosity and a dialysate flow rate.

In the case of dialysate conditions and a dialysate flow rate that are used in the evaluation tests of the following examples, a pressure loss on the dialysate side is preferably less than 20 mmHg.

A person skilled in the art can assemble a hemodiafiltration device using the hemodiafilter of the present embodiment according to common technical knowledge.

The hemodiafilter of the present embodiment can be combined with a blood flow path, a blood pump, a dialysate flow path, a dialysate feed pump, substitution fluid feed flow path, and a substitution fluid feed pump to constitute the hemodiafiltration device.

Specifically, the blood flow path is connected to the hemodiafilter, and blood is supplied to the hemodiafilter through the blood flow path by the action of the blood pump. The dialysate feed flow path is connected to the hemodiafilter, and dialysate is supplied to the hemodiafilter through the dialysate feed flow path by the action of the dialysate feed pump. Furthermore, a substitution fluid feed flow path is provided so as to branch from the dialysate feed flow path and connect to the blood flow path, and substitution fluid (dialysate) is fed to the blood flow path by the action of the substitution fluid pump.

The substitution fluid may be fed to the blood flow path preceding the hemodiafilter (pre-dilution in which the substitution fluid feed flow path is connected to the blood flow path upstream of the blood inlet) or may be fed to the blood flow path following the hemodiafilter (post-dilution in which the substitution fluid feed flow path is connected to the blood flow path downstream of the blood outlet).

In consideration of prevention of hemoconcentration, pre-dilution is preferably used. In this case, at least one substitution fluid feed flow path is connected to the blood flow path before the blood flow inlet of the hemodiafilter (upstream of the blood inlet).

<Examples>

The present invention will be more specifically described below with reference to examples and comparative examples. The present invention is not limited to the following examples. Cylindrical containers used for hemodiafilters in the examples and comparative examples are identical in inside diameter in the longitudinal direction unless otherwise specified.

[Evaluation test]

The interiors of the hemodiafilters were each cleaned with a liter of a physiological saline solution. Subsequently, the hemodiafilters were each fixed to a personal multi-use dialyzer DBG-03 (NIKKISO CO., LTD.) such that the centers of the hemodiafilters were 105 cm in height, and then connected to an extracorporeal circulation circuit.

4500-mL bovine blood containing a heparin anticoagulant as an anticoagulant was used as a blood pool. The blood kept at 37°C was circulated by the dialyzer under on-line pre-dilution hemodiafiltration conditions that the blood flow rate was 300 mL/min., the total dialysate flow rate was 600 mL/min., and the substitution fluid flow rate was 300 mL/min. when the effective membrane area A of the hemodiafilter was 2.2 m$^2$. When the effective membrane area A was not 2.2 m$^2$, the blood flow rate, the total dialysate flow rate, and the substitution fluid flow rate were set to the values proportioned to the effective membrane area.

The bovine blood was prepared so as to have a hematocrit of 32±2% and a protein concentration of 6.0 ± 0.5 g/dL. Blood from the blood flow outlet of the hemodiafilter was recirculated by putting the end of the extracorporeal circulation circuit into the blood pool, whereas the dialysate was fed on an one-pass basis. The dialyzer was fixed on the floor and the blood pool was installed so that a liquid level became 105 cm. Kindaly dialysis agent (Fuso Pharmaceutical Industries, Ltd.) was used as an artificial kidney dialysis agent.

During circulation, pressures were measured every second for 60 minutes with a data logger (GRAPHTEC Corporation) connected to a pressure detector connected to the hemodiafilter via a branched pipe attached to the vicinity of the blood inlet and outlet of a blood flow path and a pressure detector connected to the hemodiafilter via a branched pipe attached to the vicinity of the dialysate inlet and outlet of a dialysate flow path. Since an absolute value of a pressure value on the dialysate side varies depending on the height, the liquid levels of the branched pipes on the dialysate side were kept at 105 cm.

Data corrected at four pressure points (blood inlet side, blood outlet side, dialysate inlet side, dialysate outlet side) was plotted by spread sheet software EXCEL (Microsoft Corp.) with a horizontal axis indicating a circulation time (second) and a vertical axis indicating a pressure value (mmHg). A tertiary approximation was produced from 3600 points on the graph, and from the constant term of the approximation, pressures immediately after the start of circulation (Hereinafter will be referred to as "initial") were obtained. A pressure loss on the blood side and a pressure loss on the dialysate side were calculated from initial pressure values.

(Experiment 1)

[Examples 1, 2, and 3 and Comparative Examples 1 and 2]

A module was formed by inserting 13000 hollow fiber membranes with an inside diameter B of 200 μm and a thickness of 43 μm, each of which had a crimped shape, into a cylindrical container having a container inside diameter of 42.5 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 1 (Example 1).

A module was formed by inserting 13000 hollow fiber membranes with an inside diameter B of 200 μm and a thickness of 43 μm, each of which had a crimped shape, into a cylindrical container having a container inside diameter of 44.6 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 2 (Example 2).

A module was formed by inserting 13000 hollow fiber membranes with an inside diameter B of 200 μm and a thickness of 43 μm, each of which had a crimped shape, into a cylindrical container having a container inside diameter of 41.4 mm, and potting processing the hollow fiber membranes. The module had an effective length l of 266 mm and an effective membrane area A of 2.2 m$^2$. Headers were attached to each end of the cylindrical container, and then the container was sterilized with gamma irradiation (25 kGy) so as to obtain a hemodiafilter 3 (Example 3).

A module was formed by inserting 13000 hollow fiber membranes with an inside diameter B of 200 μm and a thickness of 43 μm, each of which did not have a crimped shape into a cylindrical container having a container inside diameter of 38.6 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 4 (Comparative Example 1).

A module was formed by inserting 14500 hollow fiber membranes with an inside diameter B of 185 μm and a thickness of 43 μm, each of which had a crimped shape, into a cylindrical container having a container inside diameter of 45.8 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 5 (Comparative Example 2).

In Example 1, a filling ratio F of a hollow fiber membrane bundle was 58.9%, a distance L between both end faces having an opening of the hollow fiber membrane was 286 mm, and an opening ratio X of an end face having openings of the hollow fiber membrane bundle was 23.3%.

In Example 2, a filling ratio F of a hollow fiber membrane bundle was 53.5%, a distance L between both end faces having an opening of the hollow fiber membrane was 286 mm, and an opening ratio X of an end face having openings of the hollow fiber membrane bundle was 22.8%.

In Example 3, a filling ratio F of a hollow fiber membrane bundle was 62.0%, a distance L between both end faces having an opening of the hollow fiber membrane was 286 mm, and an opening ratio X of an end face having openings of the hollow fiber membrane bundle was 23.8%.

In Comparative Example 1, a filling ratio F of a hollow fiber membrane bundle was 71.4%, a distance L between both end faces having an opening of the hollow fiber membrane was 286 mm, and an opening ratio X of an end face having openings of the hollow fiber membrane bundle was 28.3%.

In Comparative Example 2, a filling ratio F of a hollow fiber membrane bundle was 50.8%, a distance L between both end faces having an opening of the hollow fiber membrane was 286 mm, and an opening ratio X of an end face having openings of the hollow fiber membrane bundle was 20.0%.

By using the hemodiafilters according to Examples 1 to 3 that satisfy the relationship of expression (1) and Comparative Examples 1 and 2 that do not satisfy the relationship of expression (1), pressure losses were evaluated under the hemodiafiltration conditions described above. The results are shown in Table 1.

In Example 1, the initial value of a pressure loss was 138 mmHg on the blood side, whereas the initial value of a pressure loss was 14 mmHg on the dialysate side.

In Example 2, the initial value of a pressure loss was 139 mmHg on the blood side, whereas the initial value of a pressure loss was 7 mmHg on the dialysate side.

In Example 3, the initial value of a pressure loss was 138 mmHg on the blood side, whereas the initial value of a pressure loss was 19 mmHg on the dialysate side.

In the hemodiafilter of Comparative Example 1 where the filling ratio F of the hollow fiber membrane bundle was 71.7% and expression (1) was not satisfied, the initial value of a pressure loss was 139 mmHg on the blood side, whereas the initial value of a pressure loss was 34 mmHg on the dialysate side, and the initial value of a pressure loss on the dialysate side was high.

In the hemodiafilter of Comparative Example 2 where the filling ratio F of the hollow fiber membrane bundle was 50.8% and expression (1) was not satisfied, the initial value of a pressure loss was 160 mmHg on the blood side, whereas the initial value of a pressure loss was 5 mmHg on the dialysate side. This proves that the initial value of a pressure loss on the blood side was high.

[Table 1]

| | Effective membrane area A (m$^2$) | Crimp | Filling ratio F of hollow fiber membrane bundle (%) | Opening ratio X of end face having openings of hollow fiber membrane bundle (%) | Distance L between both end faces having opening (mm) | Pressure loss on blood side (mmHg) | Pressure loss on dialysate side (mmHg) |
|---|---|---|---|---|---|---|---|
| Example 1 | 2.2 | Crimped | 58.9 | 23.3 | 286 | 138 | 14 |
| Example 2 | 2.2 | Crimped | 53.5 | 22.8 | 286 | 139 | 7 |
| Example 3 | 2.2 | Crimped | 62.0 | 23.8 | 286 | 138 | 19 |
| Comparative Example 1 | 2.2 | Not crimped | 71.4 | 28.3 | 286 | 139 | 34 |
| Comparative Example 2 | 2.2 | Crimped | 50.8 | 20.0 | 286 | 160 | 5 |

[Examples 4, 5, 6, and 7 and Comparative Examples 3 and 4]

A module was formed by inserting 13000 hollow fiber membranes with an inside diameter B of 200 $\mu$m and a thickness of 43 $\mu$m, each of which had a crimped shape, into a cylindrical container having a container diameter of 43.8 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 6 (Example 4).

A module was formed by inserting 12000 hollow fiber membranes with an inside diameter B of 220 μm and a thickness of 34 μm, each of which had a crimped shape, into a cylindrical container having a container diameter of 41 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 7 (Example 5).

A module was formed by inserting 11000 hollow fiber membranes with an inside diameter B of 240 μm and a thickness of 23 μm, each of which had a crimped shape, into a cylindrical container having a container diameter of 40.7 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 8 (Example 6).

A module was formed by inserting 10500 hollow fiber membranes with an inside diameter B of 250 μm and a thickness of 30 μm, each of which had a crimped shape, into a cylindrical container having a container diameter of 40.7 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 9 (Example 7).

A module was formed by inserting 14500 hollow fiber membranes with an inside diameter B of 180 μm and a thickness of 43 μm, each of which had a crimped shape, into a cylindrical container having a container diameter of 42.5 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 10 (Comparative Example 3).

A module was formed by inserting 10500 hollow fiber membranes with an inside diameter B of 255 μm and a thickness of 43 μm, each of which did not have a crimped shape, into a cylindrical container having a container diameter of 40.7 mm, and potting processing the hollow fiber membranes. The module had an effective length l of 266 mm and an effective membrane area A of 2.2 m$^2$. Headers were attached to both ends of the cylindrical container, and then the container was sterilized with gamma irradiation (25 kGy) so as to obtain a hemodiafilter 11 (Comparative Example 4).

In Example 4, a filling ratio F of a hollow fiber membrane bundle was 55.4%, a distance L between both end faces having an opening of the hollow fiber membrane was 286 mm, and an opening ratio X of an end face having openings of the hollow fiber membrane bundle was 23.0%.

In Example 5, a filling ratio F of a hollow fiber membrane bundle was 59.2%, a distance L between both end faces having an opening of the hollow fiber membrane was 286 mm, and an opening ratio X of an end face having openings of the hollow fiber membrane bundle was 27.1%.

In Example 6, a filling ratio F of a hollow fiber membrane bundle was 54.3%, a distance L between both end faces having an opening of the hollow fiber membrane was 286 mm, and an opening ratio X of an end face having openings of the hollow fiber membrane bundle was 30.9%.

In Example 7, a filling ratio F of a hollow fiber membrane bundle was 60.9%, a distance L between both end faces having an opening of the hollow fiber membrane was 286 mm, and an opening ratio X of an end face having openings of the hollow fiber membrane bundle was 31.4%.

In Comparative Example 3, a filling ratio F of a hollow fiber membrane bundle was 56.8%, a distance L between both end faces having an opening of the hollow fiber membrane was 286 mm, and an opening ratio X of an end face having openings of the hollow fiber membrane bundle was 21.1%.

In Comparative Example 4, a filling ratio F of a hollow fiber membrane bundle was 73.7%, a distance L between both end faces having an opening of the hollow fiber membrane was 286 mm, and an opening ratio X of an end face having openings of the hollow fiber membrane bundle was 33.3%.

By using the hemodiafilters according to Examples 4 to 7 that satisfy the relationship of expression (1) and Comparative Examples 3 and 4 that do not satisfy the relationship of expression (1), pressure losses were evaluated under the hemodiafiltration conditions described above. The results are shown in Table 2.

In the hemodiafilter of Example 4, the initial value of a pressure loss was 137 mmHg on the blood side, whereas the initial value of a pressure loss was 10 mmHg on the dialysate side.

In the hemodiafilter of Example 5, the initial value of a pressure loss was 117 mmHg on the blood side, whereas the initial value of a pressure loss was 16 mmHg on the dialysate side.

In the hemodiafilter of Example 6, the initial value of a pressure loss was 101 mmHg on the blood side, whereas the initial value of a pressure loss was 9 mmHg on the dialysate side.

In the hemodiafilter of Example 7, the initial value of a pressure loss was 97 mmHg on the blood side, whereas the initial value of a pressure loss was 16 mmHg on the dialysate side.

In the hemodiafilter of Comparative Example 3 where the opening ratio X of an end face having an opening was 21.1% and the relationship of expression (1) was not satisfied, the initial value of a pressure loss was 172 mmHg on the blood side, whereas the initial value of a pressure loss was 13 mmHg on the dialysate side, and the initial value of a pressure loss on the blood side was high.

In the hemodiafilter of Comparative Example 4 where the opening ratio X of an end face having an opening was 33.3% and the relationship of expression (1) was not satisfied, the initial value of a pressure loss was 94 mmHg on the blood side, whereas the initial value of a pressure loss was 30 mmHg on the dialysate side,and the initial value of a pressure loss on the dialysate side was high.

[Table 2]

| | Effective membrane area A (m$^2$) | Crimp | Filling ratio F of hollow fiber membrane bundle (%) | Opening ratio X of end face having openings of hollow fiber membrane bundle (%) | Distance L between both end faces having opening (mm) | Pressure loss on blood side (mmHg) | Pressure loss on dialysate side (mmHg) |
|---|---|---|---|---|---|---|---|
| Example 4 | 2.2 | Crimped | 55.4 | 23.0 | 286 | 137 | 10 |
| Example 5 | 2.2 | Crimped | 59.2 | 27.1 | 286 | 117 | 16 |
| Example 6 | 2.2 | Crimped | 54.3 | 30.9 | 286 | 101 | 9 |
| Example 7 | 2.2 | Crimped | 60.9 | 31.4 | 286 | 97 | 16 |
| Comparative Example 3 | 2.2 | Crimped | 56.8 | 21.1 | 286 | 172 | 13 |
| Comparative Example 4 | 2.2 | Not crimped | 73.7 | 33.3 | 286 | 94 | 30 |

[Examples 8, 9, and 10 and Comparative Example 5]

A module was formed by inserting 17000 hollow fiber membranes with an inside diameter B of 200 $\mu$m and a thickness of 40 $\mu$m, each of which had a crimped shape, into a cylindrical container having a container diameter of 47.7 mm, and potting processing the hollow fiber membranes. The module had an effective length l of 204 mm and an effective membrane area A of 2.2 m$^2$. Headers were attached to both ends of the cylindrical container, and then the container was sterilized with gamma irradiation (25 kGy) so as to obtain a hemodiafilter 12 (Example 8).

A module was formed by inserting 14500 hollow fiber membranes with an inside diameter B of 200 $\mu$m and a thickness of 40 $\mu$m, each of which had a crimped shape, into a cylindrical container having a container diameter of 43.8 mm, and potting processing the hollow fiber membranes. The module had an effective length l of 241 mm and an effective membrane area A of 2.2 m$^2$. Headers were attached to both ends of the cylindrical container, and then the container was sterilized with gamma irradiation (25 kGy) so as to obtain a hemodiafilter 13 (Example 9).

A module was formed by inserting 11500 hollow fiber membranes with an inside diameter B of 205 $\mu$m and a thickness of 40 $\mu$m, each of which had a crimped shape, into a cylindrical container having a container diameter of 39.6 mm, and potting processing the hollow fiber membranes. The module had an effective length l of 290 mm and an effective membrane area A of 2.2 m$^2$. Headers were attached to both ends of the cylindrical container, and then the container was sterilized with gamma irradiation (25 kGy) so as to obtain a hemodiafilter 14 (Example 10).

A module was formed by inserting 10400 hollow fiber membranes with an inside diameter B of 200 $\mu$m and a thickness of 40 $\mu$m, each of which had a crimped shape, into a cylindrical container having a container diameter of 36.9 mm, and potting processing the hollow fiber membranes. The module had an effective length l of 340 mm and an effective membrane area A of 2.2 m$^2$. Headers were attached to both ends of the cylindrical container, and then the container was sterilized with gamma irradiation (25 kGy) so as to obtain a hemodiafilter 15 (Comparative Example 5).

In Example 8, a filling ratio F of a hollow fiber membrane bundle was 58.6%, a distance L between both end faces having an opening of the hollow fiber membrane was 220 mm, and an opening ratio X of an end face having openings of the hollow fiber membrane bundle was 26.1%.

In Example 9, a filling ratio F of a hollow fiber membrane bundle was 59.3%, a distance L between both end faces having an opening of the hollow fiber membrane was 257 mm, and an opening ratio X of an end face having openings of the hollow fiber membrane bundle was 25.7%.

In Example 10, a filling ratio F of a hollow fiber membrane bundle was 59.7%, a distance L between both end faces having an opening of the hollow fiber membrane was 310 mm, and an opening ratio X of an end face having openings of the hollow fiber membrane bundle was 24.7%.

In Comparative Example 5, a filling ratio F of a hollow fiber membrane bundle was 59.9%, a distance L between both end faces having an opening of the hollow fiber membrane was 360 mm, and an opening ratio X of an end face having openings of the hollow fiber membrane bundle was 23.9%.

By using the hemodiafilters according to Examples 8 to 10 that satisfy the relationship of expression (1) and Comparative Example 5 that does not satisfy the relationship of expression (1), pressure losses were evaluated under the hemodiafiltration conditions described above. The results are shown in Table 3.

In the hemodiafilter of Example 8, the initial value of a pressure loss was 104 mmHg on the blood side, whereas the initial value of a pressure loss was 9 mmHg on the dialysate side.

In the hemodiafilter of Example 9, the initial value of a pressure loss was 122 mmHg on the blood side, whereas the initial value of a pressure loss was 13 mmHg on the dialysate side.

In the hemodiafilter of Example 10, the initial value of a pressure loss was 149 mmHg on the blood side, whereas the initial value of a pressure loss was 19 mmHg on the dialysate side.

In the hemodiafilter of Comparative Example 5 where the distance L between both end faces having an opening of the hollow fiber membrane was 360 mm and the relationship of expression (1) was not satisfied, the initial value of a pressure loss was 190 mmHg on the blood side, whereas the initial value of a pressure loss was 24 mmHg on the dialysate side, and the initial values of pressure losses on both of the blood and dialysate sides were high.

[Table 3]

| | Effective membrane area A (m²) | Crimp | Filling ratio F of hollow fiber membrane bundle (%) | Opening ratio X of end face having openings of hollow fiber membrane bundle (%) | Distance L between both end faces having opening (mm) | Pressure loss on blood side (mmHg) | Pressure loss on dialysate side (mmHg) |
|---|---|---|---|---|---|---|---|
| Example 8 | 2.2 | Crimped | 58.6 | 26.1 | 220 | 104 | 9 |
| Example 9 | 2.2 | Crimped | 59.3 | 25.7 | 257 | 122 | 13 |
| Example 10 | 2.2 | Crimped | 59.7 | 24.7 | 310 | 149 | 19 |
| Comparative Example 5 | 2.2 | Crimped | 59.9 | 23.9 | 360 | 190 | 24 |

[Examples 11, 12, 13, and 14]

A module was formed by inserting 6400 hollow fiber membranes with an inside diameter B of 200 μm and a thickness of 43 μm, each of which had a crimped shape, into a cylindrical container having a container diameter of 30 mm, and potting processing the hollow fiber membranes. The module had an effective length l of 241 mm and an effective membrane area A of 1.0 m². Headers were attached to both ends of the cylindrical container, and then the container was sterilized with gamma irradiation (25 kGy) so as to obtain a hemodiafilter 16 (Example 11).

A module was formed by inserting 9500 hollow fiber membranes with an inside diameter B of 200 μm and a thickness of 43 μm, each of which had a crimped shape, into a cylindrical container having a container diameter of 36.4 mm, and potting processing the hollow fiber membranes. The module had an effective length l of 266 mm and an effective membrane area A of 1.6 m². Header were attached to both ends of the cylindrical container, and then the container was sterilized with gamma irradiation (25 kGy) so as to obtain a hemodiafilter 17 (Example 12).

A module was formed by inserting 15400 hollow fiber membranes with an inside diameter B of 200 μm and a thickness of 43 μm, each of which had a crimped shape, into a cylindrical container having a container diameter of 46 mm, and potting processing the hollow fiber membranes. The module had an effective length l of 266 mm and an effective membrane area A of 2.2 m². Headers were attached to both ends of the cylindrical container, and then the container was sterilized with gamma irradiation (25 kGy) so as to obtain a hemodiafilter 18 (Example 13).

A module was formed by inserting 19000 hollow fiber membranes with an inside diameter B of 200 μm and a thickness of 43 μm, each of which had a crimped shape, into a cylindrical container having a container diameter of 51 mm, and potting processing the hollow fiber membranes. The module had an effective length l of 266 mm and an effective membrane area A of 2.2 m². Headers were attached to both end of the cylindrical container, and then the container was sterilized with gamma irradiation (25 kGy) so as to obtain a hemodiafilter 19 (Example 14).

In Example 11, a filling ratio F of a hollow fiber membrane bundle was 58.2%, a distance L between both end faces having an opening of the hollow fiber membrane was 257 mm, and an opening ratio X of an end face having openings of the hollow fiber membrane bundle was 20.5%.

In Example 12, a filling ratio F of a hollow fiber membrane bundle was 58.6%, a distance L between both end faces having an opening of the hollow fiber membrane was 286 mm, and an opening ratio X of an end face having openings of the hollow fiber membrane bundle was 21.5%.

In Example 13, a filling ratio F of a hollow fiber membrane bundle was 59.5%, a distance L between both end faces having an opening of the hollow fiber membrane was 286 mm, and an opening ratio X of an end face having an opening of the hollow fiber membrane bundle was 24.6%.

In Example 14, a filling ratio F of a hollow fiber membrane bundle was 59.8%, a distance L between both end faces having an opening of the hollow fiber membrane was 286 mm, and an opening ratio X of an end face having openings of the hollow fiber membrane bundle was 26.2%.

By using the hemodiafilters according to Examples 11 to 14 that satisfy the relationship of expression (1), pressure losses were evaluated under the hemodiafiltration conditions described above. The results are shown in Table 4.

In the hemodiafilter of Example 11, the initial value of a pressure loss was 123 mmHg on the blood side, whereas the initial value of a pressure loss was 10 mmHg on the dialysate side.

In the hemodiafilter of Example 12, the initial value of a pressure loss was 135 mmHg on the blood side, whereas the initial value of a pressure loss was 14 mmHg on the dialysate side.

In the hemodiafilter of Example 13, the initial value of a pressure loss was 138 mmHg on the blood side, whereas the initial value of a pressure loss was 15 mmHg on the dialysate side.

In the hemodiafilter of Example 14, the initial value of a pressure loss was 136 mmHg on the blood side, whereas the initial value of a pressure loss was 14 mmHg on the dialysate side.

The initial values of pressure losses on the blood and dialysate sides of the hemodiafiltration module according to Example 11 were in the same range as those of Example 9, whereas the initial values of pressure losses on the blood and dialysate sides of the hemodiafiltration modules according to Examples 12, 13, and 14 were in the same range as those of Example 1.

[Table 4]

| | Effective membrane area A (m$^2$) | Crimp | Filling ratio F of hollow fiber membrane bundle (%) | Opening ratio X of end face having openings of hollow fiber membrane bundle (%) | Distance L between both end faces having opening (mm) | Pressure loss on blood side (mmHg) | Pressure loss on dialysate side (mmHg) |
|---|---|---|---|---|---|---|---|
| Example 11 | 1.0 | Crimped | 58.2 | 20.5 | 257 | 123 | 10 |
| Example 12 | 1.6 | Crimped | 58.6 | 21.5 | 286 | 135 | 14 |
| Example 13 | 2.6 | Crimped | 59.5 | 24.6 | 286 | 138 | 15 |
| Example 14 | 3.2 | Crimped | 59.8 | 26.2 | 286 | 136 | 14 |

(Experiment 2)

[Examples 15 to 17 and Comparative Examples 6 and 7]

In Example 15, the hemodiafilter 1 used in Example 1 was evaluated. A filling ratio F of a hollow fiber membrane bundle was 58.9%, a distance L between both end faces having an opening of a hollow fiber membrane was 286 mm, and an opening ratio Y of a blood flow path area on an end face of curing resin was 21.1%.

In Example 16, the hemodiafilter 2 used in Example 2 was evaluated. A filling ratio F of a hollow fiber membrane bundle was 53.5%, a distance L between both end faces having an opening of a hollow fiber membrane was 286 mm, and an opening ratio Y of a blood flow path area on an end face of curing resin was 20.6%.

In Example 17, the hemodiafilter 3 used in Example 3 was evaluated. A filling ratio F of a hollow fiber membrane bundle was 62.0%, a distance L between both end faces having an opening of a hollow fiber membrane was 286 mm, and an opening ratio Y of a blood flow path area on an end face of curing resin was 21.6%.

In Comparative Example 6, the hemodiafilter 4 used in Comparative Example 1 was evaluated. A filling ratio F of a hollow fiber membrane bundle was 71.4%, a distance L between both end faces having an opening of a hollow fiber membrane was 286 mm, and an opening ratio Y of a blood flow path area on an end face of curing resin was 25.3%.

In Comparative Example 7, the hemodiafilter 5 used in Comparative Example 2 was evaluated. A filling ratio F of a hollow fiber membrane bundle was 50.8%, a distance L between both end faces having an opening of a hollow fiber membrane was 286 mm, and an opening ratio Y of a blood flow path area on an end face of curing resin was 18.2%.

By using the hemodiafilters according to Examples 15 to 17 that satisfy the relationship of expression (a) and Comparative Examples 6 and 7 that do not satisfy the relationship of expression (a), pressure losses were evaluated under the hemodiafiltration conditions described above. The results are shown in Table 5.

In Example 15, the initial value of a pressure loss was 138 mmHg on the blood side, whereas the initial value of a pressure loss was 14 mmHg on the dialysate side.

In Example 16, the initial value of a pressure loss was 139 mmHg on the blood side, whereas the initial value of a pressure loss was 7 mmHg on the dialysate side.

In Example 17, the initial value of a pressure loss was 138 mmHg on the blood side, whereas the initial value of a pressure loss was 19 mmHg on the dialysate side.

In the hemodiafilter of Comparative Example 6 where the filling ratio F of the hollow fiber membrane bundle was 71.4%, the initial value of a pressure loss was 139 mmHg on the blood side, whereas the initial value of a pressure loss was 34 mmHg on the dialysate side, and the initial value of a pressure loss on the dialysate side was high.

In the hemodiafilter of Comparative Example 7 where the filling ratio F of the hollow fiber membrane bundle was 50.8%, the initial value of a pressure loss was 160 mmHg on the blood side, whereas the initial value of a pressure loss was 5 mmHg on the dialysate side, and the initial value of a pressure loss on the blood side was high.

[Table 5]

| | Effective membrane area A (m$^2$) | Crimp | Filling ratio F of hollow fiber membrane bundle (%) | Opening ratio Y of blood flow path area on end face of curing resin (%) | Distance L between both end faces having opening (mm) | Pressure loss on blood side (mmHg) | Pressure loss on dialysate side (mmHg) |
|---|---|---|---|---|---|---|---|
| Example 15 | 2.2 | Crimped | 58.9 | 21.1 | 286 | 138 | 14 |
| Example 16 | 2.2 | Crimped | 53.5 | 20.6 | 286 | 139 | 7 |
| Example 17 | 2.2 | Crimped | 62.0 | 21.6 | 286 | 138 | 19 |
| Comparative Example 6 | 2.2 | Not crimped | 71.4 | 25.3 | 286 | 139 | 34 |
| Comparative Example 7 | 2.2 | Crimped | 50.8 | 18.2 | 286 | 160 | 5 |

[Examples 18 to 20 and Comparative Examples 8 and 9]

A module was formed by inserting 14930 hollow fiber membranes with an inside diameter B of 200 μm and a thickness of 40 μm, each of which had a crimped shape, into a cylindrical container having a container diameter of 43.8 mm, and potting processing the hollow fiber membranes. The module had an effective length l of 241 mm and an effective membrane area A of 2.2 m$^2$. Headers were attached to both ends of the cylindrical container, and then the container was sterilized with gamma irradiation (25 kGy) so as to obtain a hemodiafilter 20 (Example 20).

A module was formed by inserting 14500 hollow fiber membranes with an inside diameter B of 175 μm and a thickness of 48 μm, each of which had a crimped shape, into a cylindrical container having a container diameter of 42.5 mm, and potting processing the hollow fiber membranes. The module had an effective length l of 266 mm and an effective membrane area A of 2.1 m$^2$. Header were attached to both ends of the cylindrical container, and then the container was sterilized with gamma irradiation (25 kGy) so as to obtain a hemodiafilter 21 (Comparative Example 8).

In Example 18, the hemodiafilter 6 used in Example 4 was evaluated. A filling ratio F of a hollow fiber membrane bundle was 55.4%, a distance L between both end faces having an opening of a hollow fiber membrane was 286 mm, and an opening ratio Y of a blood flow path area on an end face of curing resin was 20.9%.

In Example 19, the hemodiafilter 7 used in Example 4 was evaluated. A filling ratio F of a hollow fiber membrane bundle was 59.2%, a distance L between both end faces having an opening of a hollow fiber membrane was 286 mm, and an opening ratio Y of a blood flow path area on an end face of curing resin was 24.5%.

In Example 20, the newly obtained hemodiafilter 20 was evaluated. A filling ratio F of a hollow fiber membrane bundle was 61.0%, a distance L between both end faces having an opening of a hollow fiber membrane was 257 mm, and an opening ratio Y of a blood flow path area on an end face of curing resin was 24.0%.

In Comparative Example 8, the newly obtained hemodiafilter 21 was evaluated. A filling ratio F of a hollow fiber membrane bundle was 59.0%, a distance L between both end faces having an opening of a hollow fiber membrane was 286 mm, and an opening ratio Y of a blood flow path area on an end face of curing resin was 18.1%.

In Comparative Example 9, the hemodiafilter 11 used in Comparative Example 4 was evaluated. A filling ratio F of a hollow fiber membrane bundle was 73.7%, a distance L between both end faces having an opening of a hollow fiber membrane was 286 mm, and an opening ratio Y of a blood flow path area on an end face of curing resin was 30%.

By using the hemodiafilters according to Examples 18 to 20 that satisfy the relationship of expression (a) and Comparative Examples 8 and 9 that do not satisfy the relationship of expression (a), pressure losses were evaluated under the hemodiafiltration conditions. The results are shown in Table 6.

In the hemodiafilter of Example 18, the initial value of a pressure loss was 137 mmHg on the blood side, whereas the initial value of a pressure loss was 10 mmHg on the dialysate side.

In the hemodiafilter of Example 19, the initial value of a pressure loss was 117 mmHg on the blood side, whereas the initial value of a pressure loss was 16 mmHg on the dialysate side.

In the hemodiafilter of Example 20, the initial value of a pressure loss was 120 mmHg on the blood side, whereas the initial value of a pressure loss was 15 mmHg on the dialysate side.

In the hemodiafilter of Comparative Example 8 where the opening ratio Y of the blood flow path area on the end face of curing resin was 18.1 % and the relationship of expression (a) was not satisfied, the initial value of a pressure loss was 184 mmHg on the blood side, whereas the initial value of a pressure loss was 16 mmHg on the dialysate side, and the initial value of a pressure loss on the blood side was high.

In the hemodiafilter of Comparative Example 9 where the opening ratio Y of the blood flow path area on the end face of curing resin was 30% and the relationship of expression (a) was not satisfied, the initial value of a pressure loss was 94 mmHg on the blood side, whereas the initial value of a pressure loss was 30 mmHg on the dialysate side, and the initial value of a pressure loss on the dialysate side was high.

[Table 6]

| | Effective membrane area A (m$^2$) | Crimp | Filling ratio F of hollow fiber membrane bundle (%) | Opening ratio Y of blood flow path area on end face of curing resin (%) | Distance L between both end faces having opening (mm) | Pressure loss on blood side (mmHg) | Pressure loss on dialysate side (mmHg) |
|---|---|---|---|---|---|---|---|
| Example 18 | 2.2 | Crimped | 55.4 | 20.9 | 286 | 137 | 10 |
| Example 19 | 2.2 | Crimped | 59.2 | 24.5 | 286 | 117 | 16 |
| Example 20 | 2.2 | Crimped | 61 | 24.0 | 257 | 120 | 15 |
| Comparative Example 8 | 2.1 | Crimped | 59 | 18.1 | 286 | 184 | 16 |
| Comparative Example 9 | 2.2 | Not crimped | 73.7 | 30 | 286 | 94 | 30 |

[Examples 21 to 23 and Comparative Example 10]

In Example 21, the hemodiafilter 12 used in Example 8 was evaluated. A filling ratio F of a hollow fiber membrane bundle was 58.6%, a distance L between both end faces having an opening of a hollow fiber membrane was 220 mm, and an opening ratio Y of a blood flow path area on an end face of curing resin was 23.8%.

In Example 22, the hemodiafilter 13 used in Example 9 was evaluated. A filling ratio F of a hollow fiber membrane bundle was 59.3%, a distance L between both end faces having an opening of a hollow fiber membrane was 257 mm, and an opening ratio Y of a blood flow path area on an end face of curing resin was 23.3%.

In Example 23, the hemodiafilter 14 used in Example 10 was evaluated. A filling ratio F of a hollow fiber membrane bundle was 59.7%, a distance L between both end faces having an opening of a hollow fiber membrane was 310 mm, and an opening ratio Y of a blood flow path area on an end face of curing resin was 22.2%.

In Comparative Example 10, the hemodiafilter 15 used in Comparative Example 5 was evaluated. A filling ratio F of a hollow fiber membrane bundle was 59.9%, a distance L between both end faces having an opening of a hollow fiber membrane was 360 mm, and an opening ratio Y of a blood flow path area on an end face of curing resin was 21.4%.

By using the hemodiafilters according to Examples 21 to 23 that satisfy the relationship of expression (a) and Comparative Example 10 that does not satisfy the relationship of expression (a), pressure losses were evaluated under the hemodiafiltration conditions described above. The results are shown in Table 7.

In the hemodiafilter of Example 21, the initial value of a pressure loss was 104 mmHg on the blood side, whereas the initial value of a pressure loss was 9 mmHg on the dialysate side.

In the hemodiafilter of Example 22, the initial value of a pressure loss was 122 mmHg on the blood side, whereas the initial value of a pressure loss was 13 mmHg on the dialysate side.

In the hemodiafilter of Example 23, the initial value of a pressure loss was 149 mmHg on the blood side, whereas the initial value of a pressure loss was 19 mmHg on the dialysate side.

In the hemodiafilter of Comparative Example 10 where the distance L between both end faces having an opening of the hollow fiber membrane was 360 mm, the initial value of a pressure loss was 190 mmHg on the blood side, whereas the initial value of a pressure loss was 24 mmHg on the dialysate side. This proves that the initial value of pressure losses on the blood and dialysate sides were high initial values.

[Table 7]

| | Effective membrane area A (m²) | Crimp | Filling ratio F of hollow fiber membrane bundle (%) | Opening ratio Y of blood flow path area on end face of curing resin (%) | Distance L between both end faces having opening (mm) | Pressureloss on blood side (mmHg) | Pressure loss on dialysate side (mmHg) |
|---|---|---|---|---|---|---|---|
| Example 21 | 2.2 | Crimped | 58.6 | 23.8 | 220 | 104 | 9 |
| Example 22 | 2.2 | Crimped | 59.3 | 23.3 | 257 | 122 | 13 |
| Example 23 | 2.2 | Crimped | 59.7 | 22.2 | 310 | 149 | 19 |
| Comparative Example 10 | 2.2 | Crimped | 59.9 | 21.4 | 360 | 190 | 24 |

[Examples 24 to 27]

In Example 24, the hemodiafilter 16 used in Example 11 was evaluated. A filling ratio F of a hollow fiber membrane bundle was 58.2%, a distance L between both end faces having an opening of a hollow fiber membrane was 257 mm, and an opening ratio Y of a blood flow path area on an end face of curing resin was 19.4%.

In Example 25, the hemodiafilter 17 used in Example 12 was evaluated. A filling ratio F of a hollow fiber membrane bundle was 58.6%, a distance L between both end faces having an opening of a hollow fiber membrane was 286 mm, and an opening ratio Y of a blood flow path area on an end face of curing resin was 20.0%.

In Example 26, the hemodiafilter 18 used in Example 13 was evaluated. A filling ratio F of a hollow fiber membrane bundle was 59.5%, a distance L between both end faces having an opening of a hollow fiber membrane was 286 mm, and an opening ratio Y of a blood flow path area on an end face of curing resin was 22.3%.

In Example 27, the hemodiafilter 19 used in Example 14 was evaluated. A filling ratio F of a hollow fiber membrane bundle was 59.8%, a distance L between both end faces having an opening of a hollow fiber membrane was 286 mm, and an opening ratio Y of a blood flow path area on an end face of curing resin was 24.0%.

By using the hemodiafilters according to Examples 24 to 27 that satisfy the relationship of expression (a), pressure losses were evaluated under the hemodiafiltration conditions described above. The results are shown in Table 8.

In the hemodiafilter of Example 24, the initial value of a pressure loss was 123 mmHg on the blood side, whereas the initial value of a pressure loss was 10 mmHg on the dialysate side.

In the hemodiafilter of Example 25, the initial value of a pressure loss was 135 mmHg on the blood side, whereas the initial value of a pressure loss was 14 mmHg on the dialysate side.

In the hemodiafilter of Example 26, the initial value of a pressure loss was 138 mmHg on the blood side, whereas the initial value of a pressure loss was 15 mmHg on the dialysate side.

In the hemodiafilter of Example 27, the initial value of a pressure loss was 136 mmHg on the blood side, whereas the initial value of a pressure loss was 14 mmHg on the dialysate side.

The initial values of pressure losses on the blood and dialysate sides of the hemodiafiltration module according to Example 24 were in the same range as those of Example 22, whereas the initial values of pressure losses on the blood and dialysate sides of the hemodiafiltration modules according to Examples 25, 26, and 27 were in the same range as those of Example 15.

[Table 8]

| | Effective membrane area A (m²) | Crimp | Filling ratio F of hollow fiber membrane bundle (%) | Opening ratio Y of blood flow path area on end face of curing resin (%) | Distance L between both end faces having opening (mm) | Pressure loss on blood side (mmHg) | Pressureloss on dialysate side (mmHg) |
|---|---|---|---|---|---|---|---|
| Example 24 | 1.0 | Crimped | 58.2 | 19.4 | 257 | 123 | 10 |
| Example 25 | 1.6 | Crimped | 58.6 | 20 | 286 | 135 | 14 |
| Example 26 | 2.6 | Crimped | 59.5 | 22.3 | 286 | 138 | 15 |
| Example 27 | 3.2 | Crimped | 59.8 | 24 | 286 | 136 | 14 |

(Experiment 3)

[Examples 28 to 32]

A module was formed by inserting 13430 hollow fiber membranes with an inside diameter B of 200 $\mu$m and a thickness of 43 $\mu$m, each of which had a crimped shape, into a cylindrical container having a container diameter of 42.5 mm, and potting processing the hollow fiber membranes. The module had an effective length l of 266 mm and an effective membrane area A of 2.2 m². Headers were attached to both ends of the cylindrical container, and then the container was sterilized with gamma irradiation (25 kGy) so as to obtain a hemodiafilter 22 (Example 28).

A module was formed by inserting 13430 hollow fiber membranes with an inside diameter B of 200 $\mu$m and a thickness of 43 $\mu$m, each of which had a crimped shape, into a cylindrical container with a container diameter of 43 mm, and potting processing the hollow fiber membranes. The container had a longitudinally asymmetric shape with a minimum container diameter (inside diameter) of 42.5 mm at the position of 10 mm from the dialysate inlet side end of the body. The module had an effective length l of 266 mm and an effective membrane area A of 2.2 m². Headers were attached to both ends of the cylindrical container, and then the container was sterilized with gamma irradiation (25 kGy) so as to obtain a hemodiafilter 23 (Example 29).

A module was formed by inserting 13400 hollow fiber membranes with an inside diameter B of 200 $\mu$m and a thickness of 30 $\mu$m, each of which had a crimped shape, into a cylindrical container having a container diameter of 38.8 mm, and potting processing the hollow fiber membranes. The module had an effective length l of 266 mm and an effective membrane area A of 2.2 m². Headers were attached to both ends of the cylindrical container, and then the container was sterilized with gamma irradiation (25 kGy) so as to obtain a hemodiafilter 24 (Example 30).

A module was formed by inserting 13400 hollow fiber membranes with an inside diameter B of 200 $\mu$m and a thickness of 30 $\mu$m, each of which had a crimped shape, into a cylindrical container with a container diameter of 39.3 mm, and potting processing the hollow fiber membranes. The container had a longitudinally asymmetric shape with a minimum container diameter (inside diameter) of 38.8 mm at the position of 30 mm from the dialysate inlet side end of the body. The module had an effective length l of 266 mm and an effective membrane area A of 2.2 m². Headers were attached to both end of the cylindrical container, and then the container was sterilized with gamma irradiation (25 kGy) so as to obtain a hemodiafilter 25 (Example 31).

A module was formed by inserting 13400 hollow fiber membranes with an inside diameter B of 200 $\mu$m and a

thickness of 30 $\mu$m, each of which had a crimped shape, into a cylindrical container with a container diameter of 39.2 mm, and potting processing the hollow fiber membranes. The container had a longitudinally asymmetric shape with a minimum container diameter (inside diameter) of 38.7 mm at the position of 40 mm from the dialysate inlet side end of the body. The module had an effective length l of 266 mm and an effective membrane area A of 2.2 m$^2$. Header were attached to both ends of the cylindrical container, and then the container was sterilized with gamma irradiation (25 kGy) so as to obtain a hemodiafilter 26 (Example 32).

In Example 28, the newly obtained hemodiafilter 22 was evaluated. A filling ratio F of a hollow fiber membrane bundle was 60.8%, a distance L between both end faces having an opening of a hollow fiber membrane was 286 mm, an opening ratio X of the end face having openings was 24.1%, and an opening ratio Y of a blood flow path area on an end face of curing resin was 21.8%.

In Example 29, the newly obtained hemodiafilter 23 was evaluated. A filling ratio F of a hollow fiber membrane bundle was 57.8% (a filling ratio G at the position having a minimum inside diameter (= the percentage of the total cross-sectional area of hollow fiber membrane portions/the cross-sectional area of the cylindrical container) was 60.8%), a distance L between both end faces having an opening of a hollow fiber membrane was 286 mm, an opening ratio X of the end face having openings was 24.1%, and an opening ratio Y of a blood flow path area on an end face of curing resin was 21.8%.

In Example 30, the newly obtained hemodiafilter 24 was evaluated. A filling ratio F of a hollow fiber membrane bundle was 59.9%, a distance L between both end faces having an opening of a hollow fiber membrane was 286 mm, an opening ratio X of the end face having openings was 28.8%, and an opening ratio Y of a blood flow path area on an end face of curing resin was 25.9%.

In Example 31, the newly obtained hemodiafilter 25 was evaluated. A filling ratio F of a hollow fiber membrane bundle was 58.4% (a filling ratio G at the position having a minimum inside diameter (= the percentage of the total cross-sectional area of hollow fiber membrane portions/the cross-sectional area of the cylindrical container) was 59.9%), a distance L between both end faces having an opening of a hollow fiber membrane was 286 mm, an opening ratio X of the end face having openings was 28.8%, and an opening ratio Y of a blood flow path area on an end face of curing resin was 25.9%.

In Example 32, the newly obtained hemodiafilter 26 was evaluated. A filling ratio F of a hollow fiber membrane bundle was 58.7% (a filling ratio G at the position having a minimum inside diameter (= the percentage of the total cross-sectional area of hollow fiber membrane portions/the cross-sectional area of the cylindrical container) was 60.2%), a distance L between both end faces having an opening of a hollow fiber membrane was 286 mm, an opening ratio X of the end face having openings was 28.8%, and an opening ratio Y of a blood flow path area on an end face of curing resin was 25.9%.

Pressure losses were evaluated under the hemodiafiltration conditions described above by using the hemodiafilters according to Examples 28 to 32 that satisfy the relationships of expressions (1) and (a). The results are shown in Table 9.

In the hemodiafilter of Example 28, the initial value of a pressure loss was 137 mmHg on the blood side, whereas the initial value of a pressure loss was 16.7 mmHg on the dialysate side.

In the hemodiafilter of Example 29, the initial value of a pressure loss was 137 mmHg on the blood side, whereas the initial value of a pressure loss was 16.2 mmHg on the dialysate side.

In the case of the cylindrical container that decreases once and then increases in terms of inside diameter from the dialysate inlet to the dialysate outlet in the longitudinal direction, when the position having the minimum inside diameter was located at the dialysate inlet side from the halfway point between the dialysate inlet and the dialysate outlet, that is, the container had a longitudinally asymmetric shape (hemodiafilter 39), a pressure loss on the dialysate side was lower than that of when the position having the minimum inside diameter was located at the halfway poing between the dialysate inlet and the dialysate outlet, that is, a longitudinally symmetric shape (hemodiafiltration 38).

In the hemodiafilter of Example 30, the initial value of a pressure loss was 135 mmHg on the blood side, whereas the initial value of a pressure loss was 19.8 mmHg on the dialysate side.

In the hemodiafilter of Example 31, the initial value of a pressure loss was 135 mmHg on the blood side, whereas the initial value of a pressure loss was 18.8 mmHg on the dialysate side.

In the hemodiafilter of Example 32, the initial value of a pressure loss was 135 mmHg on the blood side, whereas the initial value of a pressure loss was 18.6 mmHg on the dialysate side.

In the case of the cylindrical container that decreases once and then increases in terms of inside diameter from the dialysate inlet to the dialysate outlet in the longitudinal direction, when the position having the minimum inside diameter was located at the dialysate inlet side from the halfway point between the dialysate inlet and the dialysate outlet, that is, the container had a longitudinally asymmetric shape (hemodiafilter 41), a pressure loss on the dialysate side was lower than that of when the position having the minimum inside diameter was located at the halfway point between the dialysate inlet and the dialysate outlet, that is, a longitudinally symmetric shape (hemodiafiltration 40).

[Table 9]

| | Minimum container diameter of body (mm) | Maximum container diameter of body (mm) | Body length (mm) | Position of minimum container diameter of body (mm) | Effective membrane area A (m$^2$) | Crimp | Filling ratio F of hollow fiber membrane bundle (%) | Maximum filling ratio G of hollow fiber membrane bundle (%) | L2/L3 | Taper "a"(%) | Opening ratio X of end face having openings of hollow fiber membrane bundle (%) | Opening ratio Y of blood flow path area on end face of curing resin (%) | Distance L between both end faces having opening (mm) | Pressure loss on blood side (mmHg) | Pressure loss on dialysate side (mmHg) |
|---|---|---|---|---|---|---|---|---|---|---|---|---|---|---|---|
| Example 28 | 42.5 | 44.4 | 227 | Halfway point | 2.2 | Crimped | 60.8 | 60.8 | 0.50 | 1.5 | 24.1 | 21.8 | 286 | 137 | 16.7 |
| Example 29 | 42.5 | 44.4 | 227 | 10 mm from body end | 2.2 | Crimped | 57.8 | 60.8 | 0.96 | 0.78 | 24.1 | 21.8 | 286 | 137 | 16.2 |
| Example 30 | 38.8 | 40.7 | 227 | Halfway point | 2.2 | Crimped | 59.9 | 59.9 | 0.50 | 1.68 | 28.8 | 25.9 | 286 | 135 | 19.8 |
| Example 31 | 38.8 | 40.7 | 227 | 30 mm from body end | 2.2 | Crimped | 58.4 | 59.9 | 0.87 | 0.96 | 28.8 | 25.9 | 286 | 135 | 18.8 |
| Example 32 | 38.7 | 41.0 | 227 | 40 mm from body end | 2.2 | Crimped | 58.7 | 60.2 | 0.82 | 1.25 | 28.8 | 25.9 | 286 | 135 | 18.6 |

Industrial Applicability

The hemodiafilter of the present invention can be used for various kinds of hemodiafiltration.

The present application is based on a Japanese patent application (Japanese Patent Application No. 2014-262758) filed to the JPO on December 25, 2014 and the contents of the application are incorporated herein by reference.

**Claims**

1. A hemodiafilter comprising a cylindrical container and a bundle of hollow fiber membranes packaged in the cylindrical container,
   wherein both ends of the hollow fiber membranes are potting processed at both ends of the cylindrical container with curing resin, and have end faces having an opening on both ends of the cylindrical container,
   the hollow fiber membrane has a crimped shape,
   a filling ratio F(%) of the hollow fiber membrane bundle in the cylindrical container is 53% or more and 63% or less,
   a distance L(mm) between both end faces having an opening of the hollow fiber membrane is 325 mm or less, and
   an effective membrane area A ($m^2$) of the hollow fiber membrane bundle and an opening ratio X(%) of an end face having openings of the hollow fiber membrane bundle satisfy expression (1) below:

$$18 + 2 \times A < X < 28 + 2 \times A \qquad (1)$$

2. A hemodiafilter comprising a cylindrical container and a bundle of hollow fiber membranes packaged in the cylindrical container,
   wherein both ends of the hollow fiber membranes are potting processed at both ends of the cylindrical container with curing resin, and have end faces having an opening on both ends of the cylindrical container,
   the hollow fiber membrane has a crimped shape,
   a filling ratio F(%) of the hollow fiber membrane bundle in the cylindrical container is 53% or more and 63% or less,
   a distance L(mm) between both end faces having an opening of the hollow fiber membrane is 325 mm or less, and
   the filling ratio F(%) of the hollow fiber membrane bundle and an opening ratio Y(%) of a blood flow path area on an end face of the curing resin satisfy expression (a) below:

$$6.4 + 0.2 \times F < Y < 14.4 + 0.2 \times F \qquad (a)$$

3. The hemodiafilter according to claim 1 or 2, wherein the effective membrane area A is 1.6 $m^2$ or more.

4. The hemodiafilter according to any one of claims 1 to 3, wherein an inside diameter of the hollow fiber membrane B is 195 $\mu$m or more and 205 $\mu$m or less.

5. The hemodiafilter according to any one of claims 1 to 4, wherein a ratio L/D is 5.5 or more and 9.0 or less where L(mm) is a distance between both end faces having an opening of the hollow fiber membrane bundle and D(mm) is an inside diameter of the cylindrical container.

6. The hemodiafilter according to any one of claims 1 to 5, wherein the cylindrical container has a dialysate inlet and a dialysate outlet, and
   the cylindrical container has a portion where the inside diameter of the cylindrical container D increases in a longitudinal direction from the dialysate inlet to the dialysate outlet.

7. The hemodiafilter according to any one of claims 1 to 6, wherein the cylindrical container has the dialysate inlet and the dialysate outlet, and
   the cylindrical container has a portion where the inside diameter of the cylindrical container D decreases in the longitudinal direction from the dialysate inlet to the dialysate outlet.

8. The hemodiafilter according to any one of claims 1 to 7, wherein the inside diameter of the cylindrical container once decreases and then increases in the longitudinal direction from the dialysate inlet to the dialysate outlet.

9. The hemodiafilter according to any one of claims 1 to 8, wherein an average taper "a" of the inside diameter of the cylindrical container in the longitudinal direction at the dialysate outlet side is from 0.7 to 1.5%.

10. The hemodiafilter according to any one of claims 6 to 9, wherein the position where the inside diameter of the cylindrical container is at a minimum is located at the dialysate inlet side from a halfway point between the dialysate inlet and the dialysate outlet.

11. The hemodiafilter according to any one of claims 8 to 10, wherein a ratio L2/L3 is from 0.5 to 0.99 where L2 is a length from a dialysate outlet side end of a body of the cylindrical container to a point where the inside diameter is at a minimum, and L3 is a length of the body of the cylindrical container.

12. The hemodiafilter according to any one of claims 6 to 11, wherein the cylindrical container further comprises a blood inlet and a blood outlet, the blood outlet being disposed at the dialysate inlet side, and the blood inlet being disposed at the dialysate outlet side in the longitudinal direction.

13. A hemodiafiltration device comprising:

the hemodiafilter according to any one of claims 1 to 12;
a blood flow path and a dialysate feed flow path that are connected to the hemodiafilter; and
a substitution fluid feed flow path branching from the dialysate feed flow path,
wherein the substitution fluid feed flow path is connected to the blood flow path upstream of the blood inlet and/or downstream of the blood outlet of the hemodiafilter.

14. The hemodiafiltration device according to claim 13, further comprising:

a blood pump that allows blood to flow into the hemodiafilter through the blood flow path;
a dialysate feed pump that supplies dialysate into the hemodiafilter through the dialysate feed flow path; and
a substitution fluid pump that feeds a substitution fluid to the blood flow path through the substitution fluid feed flow path.

[Fig 1]

[Fig 2]

**INTERNATIONAL SEARCH REPORT**

| | International application No. |
|---|---|
| | PCT/JP2015/086356 |

A. CLASSIFICATION OF SUBJECT MATTER
*A61M1/18*(2006.01)i, *B01D61/28*(2006.01)i, *B01D63/00*(2006.01)i, *B01D63/02*
(2006.01)i

According to International Patent Classification (IPC) or to both national classification and IPC

B. FIELDS SEARCHED

Minimum documentation searched (classification system followed by classification symbols)
A61M1/18, B01D61/28, B01D63/00, B01D63/02

Documentation searched other than minimum documentation to the extent that such documents are included in the fields searched
Jitsuyo Shinan Koho          1922–1996    Jitsuyo Shinan Toroku Koho    1996–2016
Kokai Jitsuyo Shinan Koho    1971–2016    Toroku Jitsuyo Shinan Koho    1994–2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2006/024902 A1  (Asahi Kasei Medical Co.,<br>Ltd.),<br>09 March 2006 (09.03.2006),<br>paragraphs [0001], [0033], [0054], [0060];<br>table 1<br>& US 2008/0237127 A1<br>paragraphs [0001], [0085], [0123] to [0126],<br>[0133]; table 1<br>& JP 4992106 B2           & EP 1790364 A1<br>& CN 101035576 A | 1–12<br>1–14 |

| ☒ Further documents are listed in the continuation of Box C. | ☐ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>    17 March 2016 (17.03.16) | Date of mailing of the international search report<br>    29 March 2016 (29.03.16) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2015/086356

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | WO 2004/094047 A1 (Asahi Medical Co., Ltd.),<br>04 November 2004 (04.11.2004),<br>page 1, lines 11 to 13; page 2, lines 8 to 10;<br>page 6, lines 20 to 24; page 14, lines 11 to<br>21; page 15, lines 2 to 6; table 1<br>& US 2007/0007193 A1<br>paragraphs [0002], [0006], [0037], [0086] to<br>[0087], [0092]; table 1<br>& JP 4678776 B2        & EP 1634639 A1<br>& CN 1777471 A | 1-12<br>1-14 |
| Y | JP 2000-042099 A (Terumo Corp.),<br>15 February 2000 (15.02.2000),<br>paragraphs [0001], [0021] to [0022], [0024];<br>table 1<br>& EP 968730 A1<br>paragraphs [0001], [0037] to [0044], [0046] to<br>[0047]; table 1 | 1-14 |
| Y | JP 2011-509131 A (Fresenius Medical Care<br>Deutschland GmbH),<br>24 March 2011 (24.03.2011),<br>fig. 1<br>& US 2010/0276367 A1<br>fig. 1<br>& WO 2009/087092 A1     & CN 101909671 A | 13-14 |
| A | JP 08-192031 A (Terumo Corp.),<br>30 July 1996 (30.07.1996),<br>entire text; all drawings<br>& US 5700372 A           & EP 701826 A2 | 1-14 |
| A | JP 2001-309974 A (Toyobo Co., Ltd.),<br>06 November 2001 (06.11.2001),<br>entire text; all drawings<br>(Family: none) | 1-14 |
| A | JP 10-305218 A (Nikkiso Co., Ltd.),<br>17 November 1998 (17.11.1998),<br>entire text; all drawings<br>(Family: none) | 1-14 |

Form PCT/ISA/210 (continuation of second sheet) (January 2015)

**EP 3 238 758 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009078121 A
- JP 4992104 B
- JP 2014262758 A